(19) Europäisches Patentamt
European Patent Office
Office européen des brevets

(11) Publication number: **0 552 186 B1**

(12) **EUROPEAN PATENT SPECIFICATION**

(45) Date of publication of patent specification: **11.01.95**

(51) Int. Cl.[6]: **C07C 69/75**, A61K 31/215

(21) Application number: **91917124.9**

(22) Date of filing: **02.10.91**

(86) International application number:
**PCT/EP91/01881**

(87) International publication number:
**WO 92/06947 (30.04.92 92/10)**

(54) **MENTHOL DERIVATIVE HAVING SPASMOLYTIC ACTIVITY.**

(30) Priority: **10.10.90 IT 216979**

(43) Date of publication of application:
**28.07.93 Bulletin 93/30**

(45) Publication of the grant of the patent:
**11.01.95 Bulletin 95/02**

(84) Designated Contracting States:
**AT BE CH DE DK ES FR GB GR IT LI LU NL SE**

(56) References cited:

**CHEMICAL ABSTRACTS, vol. 107, no. 17, 26 October 1987, Columbus, Ohio, US; PERNG, TONG HSIUNG et al.: "The insecticidal activity of n-alkyl acyl derivatives of (-)-menth and t-butyl alcohols against Sitophilus zeamais L", see abstract 149168w**

(73) Proprietor: **PULITZER ITALIANA S.R.L.**
**Via Tiburtina, 1004**
**I-00156 Rome (IT)**

(72) Inventor: **RAINOLDI, Angelo**
**Via Tiburtina, 1004**
**I-00156 Rome (IT)**

(74) Representative: **Minoja, Fabrizio**
**Studio Consulenza Brevettuale,**
**Via Rossini, 8**
**I-20122 Milano (IT)**

EP 0 552 186 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to 1-menthyl heptanoate of formula

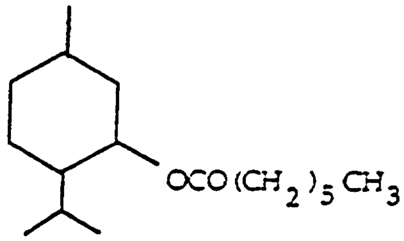

as a therapeutic agent and pharmaceutical composition containing it.

The compound used in the present invention, which will also be named PU 2054 is known as insecticide (see Chem. Abs., vol 107, no. 17, 26 October 1987). It has a potent calcium antagonist activity which appears as a remarkable long lasting spasmolytic effect both in vitro and in vivo, particularly in the intestinal tract: therefore, PU 2054 is particularly useful for the treatment of irritable colon.

PU 2054 has a spasmolytic effect on guinea pig and rabbit isolated intestine, which had previously been stimulated by agonists, such as acetylcholine, barium chloride, histamine and serotonine. PU 2054 resulted more active than papaverine.

Furthermore, PU 2054 was tested to evaluate the specificity towards calcium receptors and it proved to be particularly effective in displacing 3H-nitrendipine from intestinal smooth-muscle receptors and less active on skeletal and cardiac muscles. These findings indicate that PU 2054 calcium antagonist activity is mainly directed towards calcium receptors of intestinal muscle: accordingly, a therapeutic efficacy, together with a very low occurrence of side effects, can be foreseen.

PU 2054 spasmolytic activity was evidenced in the following in vivo tests.

a) Contractions induced by barium chloride in rabbit colon

A hemi-isochoric force transducer was applied to the colon of male New Zealand rabbits (2.5-3.0 kg), which were anesthetized with sodium pentobarbital. Colon spasms were induced by 4 mg/kg of barium chloride i.v.. PU 2054 was locally administered by transparietal injection at 2-4-8 mg/kg doses.

Contractions, registered before and after PU 2054 administration, were evaluated with a planimeter and compared each other. The animals with at least a 50% contraction inhibition were considered protected.

Results are reported in Table 1, which evidences that PU 2054 has an effective dose-related spasmolytic effect, with a ED50 of 5.88 mg/kg (confidence limits 3.37-10.26).

**Table 1**

In vivo spasmolytic activity in rabbit colon

Contractions induced by barium chloride (4 mg/kg i.v.)

| Treatment | Dose mg/kg | No. of tests | Protected animals (%) | ED50 * (mg/kg) | Confidence limits P=0.05 |
|---|---|---|---|---|---|
| PU 2054 | 2 | 10 | 10 | | |
| | 4 | 10 | 40 | 5.88 | 3.37-10.26 |
| | 8 | 10 | 60 | | |

(*) Litchfield J.T., Wilcoxon F.-J. Pharmac. Exp. Ther. 96, 99; 1949

b) Contractions induced by vagus nerve stimulation in rabbit duodenum

A suitable water-filled small ball, connected with a pressure transducer, was introduced into duodenum of male New Zealand rabbits (2.5-3.0 kg), previously anesthetized with a urethane-chloralose mixture.

Contractions induced by electric stimulation (20 Hz, 7V, 0.7 msec) of the two previously isolated and sectioned vagus nerves for 30 seconds, were evaluated with a planimeter. The contractions obtained after intravenous administrations of 0.25, 0.5, 1.0, 2.0 mg/kg of PU 2054 were compared with the basal ones.

The animals with at least a 50% contraction inhibition were considered protected.

The results reported in Table 2 show that pin 2054 inhibits spasms induced by electric stimulation with a dose-related efficacy. ED50 was 0.53 mg/kg i.v. (confidence limits 0.32-0.87).

**Table 2**

In vivo spasmolytic activity in rabbit duodenum

Contractions induced by electrically stimulated vagus nerve

| Treatment | Dose mg/kg i.v. | No. of tests | Protected animals (%) | ED50 * (mg/kg) | Confidence limits P=0.05 |
|---|---|---|---|---|---|
| PU 2054 | 0.25 | 10 | 10 | | |
| | 0.5 | 10 | 50 | | |
| | | | | 0.53 | 0.32-0.87 |
| | 1.0 | 10 | 80 | | |
| | 2.0 | 10 | 100 | | |

(*) Litchfield J.T., Wilcoxon F.-J. Pharmac. Exp. Ther. 96, 99; 1949

Further, PU 2054 spasmolytic activity was long lasting.

The present invention also relates to pharmaceutical compositions containing PU 2054 as the active ingredient. Said compositions are suitably formulated for the oral, rectal or parenteral administrations, according to conventional excipients and techniques like those described in "Remington's Pharmaceutical Sciences Handbook", Mack Pub. Co., NY, USA.

According to the present invention, the pharmaceutical compositions contain from 1 to 500 mg of PU 2054 and can be administered 1-4 times a day, according to patient's conditions.

Examples of said compositions include capsules, tablets, sugar coated pills, sustained-release forms, syrups, drops, suppositories, injectable ampoules or vials.

**Claims**

1. Menthyl heptanoate as a therapeutic agent.

2. Pharmaceutical compositions containing menthyl heptanoate as the active ingredient, in admixture with suitable carriers.

3. The use of menthyl heptanoate for the manufacturing of a medicament with spasmolytic and calcium antagonist activities.

**Patentansprüche**

1. Menthyl-Önanthat als therapeutisches Heilmittel.

2. Pharmazeutische Zusammensetzungen, welche Menthyl-Önanthat als aktiven Bestandteil beinhalten, in Beimischung mit geeigneten Trägerstoffen.

3. Anwendung von Menthyl-Önanthat zur Herstellung eines Medikamentes mit spasmolytischen und Kalzium entgegenwirkenden Wirksamkeiten.

**Revendications**

1. Heptanoate de menthyle comme agent thérapeutique.

2. Compositions pharmaceutiques contenant de l'heptanoate de menthyle comme ingrédient actif, en mélange avec des supports appropriés.

3. Utilisation de l'heptanoate de menthyle pour la fabrication d'un médicament présentant des activités spasmolytiques et antagonistes du calcium.